# EUROPEAN PATENT APPLICATION

(11) **EP 4 691 437 A1**
(43) Date of publication of application: **11.02.2026**
(21) Application number: 23930785.3
(22) Date of filing: 23.10.2023
(51) Int. Cl.: A61F 13/15

(54) **INDIVIDUALLY PACKAGED ABSORBENT ARTICLE**

(30) Priority: 30.03.2023 JP 2023055947
(71) Applicant: DAIO PAPER CORPORATION, Shikokuchuo-shi Ehime 799-0492 (JP)
(72) Inventor: WATANABE, Megumi, Sakura-shi, Tochigi 329-1411 (JP); KURAMOCHI, Mihoko, Sakura-shi, Tochigi 329-1411 (JP); ITO, Rina, Sakura-shi, Tochigi 329-1411 (JP); IWABUCHI, Haruka, Sakura-shi, Tochigi 329-1411 (JP); TAKAHASHI, Hiromi, Sakura-shi, Tochigi 329-1411 (JP)
(74) Representative: Epping - Hermann - Fischer
(86) International application number: PCT/JP2023/038201
(87) International publication number: WO 2024/202155

(57) **Abstract**

In an individually packaged absorbent article formed by individually packaging an absorbent article with a packaging sheet made of paper, the packaging sheet is folded to an inner surface side in a first direction of the packaging sheet, both edge portions in a second direction orthogonal to the first direction are sealed, and a strength improving layer is provided on an inner surface and/or an outer surface of the packaging sheet.

## Description

### TECHNICAL FIELD

The present invention relates to individually packaged absorbent articles.

### BACKGROUND ART

Many absorbent articles, such as sanitary napkins, panty liners, incontinence pads, and the like, are provided as individually packaged absorbent articles (individual packages) in a state of being individually packaged and sealed with a packaging sheet for reasons of hygiene during storage, convenience during carrying, and the like. As a packaging structure of the individually packaged absorbent article, for example, a form in which the absorbent article is arranged on the inner surface of the packaging sheet, the packaging sheet is folded to the inner surface side together with the absorbent article, and then both edge portions are sealed is widely known.

As the material of the packaging sheet, a resin film, a nonwoven fabric, or the like is still mainly used, but a packaging sheet made of paper is also known (for example, Patent Document 1).

### RELATED ART DOCUMENTS

### PATENT DOCUMENT

Patent Document 1: Japanese Unexamined Patent Publication No. 2022-24624

### SUMMARY OF THE INVENTION

### PROBLEMS TO BE SOLVED BY THE INVENTION

As a packaging sheet of an individually packaged absorbent article, it is desirable to use paper having a relatively small basis weight in consideration of ease of folding at the time of production, texture, and the like. However, since paper having a small basis weight has low strength, it may be torn in the case where a force is locally applied at the time of unsealing the individually packaged absorbent article. In this case, there is a possibility that the packaging sheet cannot be unfolded at once, which may increase the time and effort for unsealing the packaging sheet, or the packaging sheet might become unusable to wrap a used absorbent article.

In view of the above, an object of one aspect of the present invention is to prevent damage to a packaging sheet made of paper for an individually packaged absorbent article using the packaging sheet.

### MEANS FOR SOLVING THE PROBLEMS

One aspect of the present invention is an individually packaged absorbent article in which an absorbent article is individually packaged by a packaging sheet made of paper, wherein the packaging sheet is folded to an inner surface side in a first direction of the packaging sheet, both edge portions in a second direction orthogonal to the first direction are sealed, and a strength improving layer is provided on an inner surface and/or an outer surface of the packaging sheet.

### EFFECTS OF THE INVENTION

According to one aspect of the present invention, a packaging sheet made of paper for an individually packaged absorbent article using the packaging sheet can be prevented from being damaged. The configuration is obtained.

### BRIEF DESCRIPTION OF THE DRAWINGS

[FIG. 1] FIG. 1 is a plan view of an individually packaged absorbent article according to a first embodiment of the present invention.
[FIG. 2] FIG. 2 is a developed plan view of the individually packaged absorbent article of FIG. 1.
[FIG. 3] FIG. 3 is a sectional view taken along line I-I of FIG. 1.
[FIG. 4] FIG. 4 is a sectional view taken along line II-II of FIG. 1.
[FIG. 5] FIG. 5 is a view illustrating a modified example of an arrangement of a strength improving layer in the first embodiment.
[FIG. 6] FIG. 6 is a view illustrating another modified example of an arrangement of a strength improving layer in the first embodiment.
[FIG. 7] FIG. 7 is a plan view of an individually packaged absorbent article according to a second embodiment of the present invention.
[FIG. 8] FIG. 8 is a plan view of the individually packaged absorbent article of FIG. 7 in a state where a packaging sheet is unfolded.
[FIG. 9] FIG. 9 is a sectional view taken along line III-III of FIG. 7.
[FIG. 10] FIG. 10 is a plan view illustrating a state where a packaging sheet for an individually packaged absorbent article according to a third embodiment of the present invention is unfolded.
[FIG. 11] FIG. 11 is a view illustrating a modified example of a strength improving layer in the third embodiment.
[FIG. 12] FIG. 12 is a plan view illustrating a state where a packaging sheet in an individually packaged absorbent article according to a fourth embodiment of the present invention is unfolded.
[FIG. 13] FIG. 13 is a view illustrating a modified example of a strength improving layer in the fourth embodiment.
[FIG. 14] FIG. 14 is a view illustrating another modified example of a strength improving layer in the fourth embodiment.
[FIG. 15] FIG. 15 is a view illustrating yet another modified example of a strength improving layer in the fourth embodiment.

### DESCRIPTION OF EMBODIMENTS

Hereinafter, embodiments of the present invention will be described with reference to the drawings. In the drawings, the same or corresponding components are denoted by the same reference numerals unless otherwise specified, and the description thereof may be omitted.

### <First Embodiment>

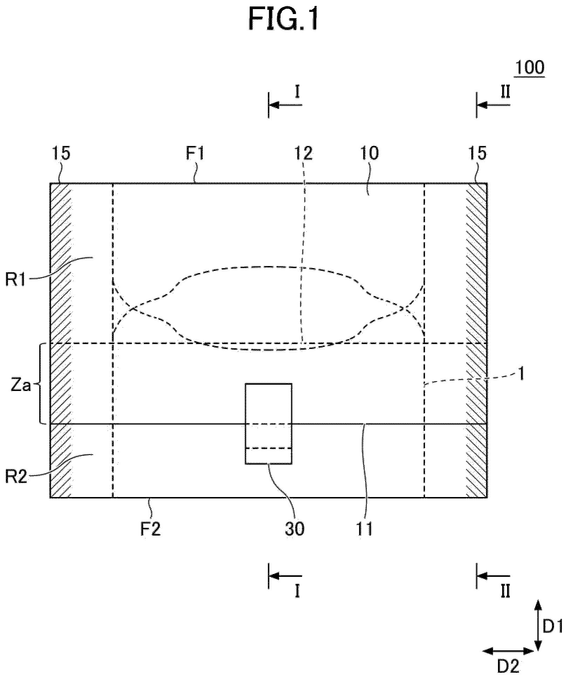

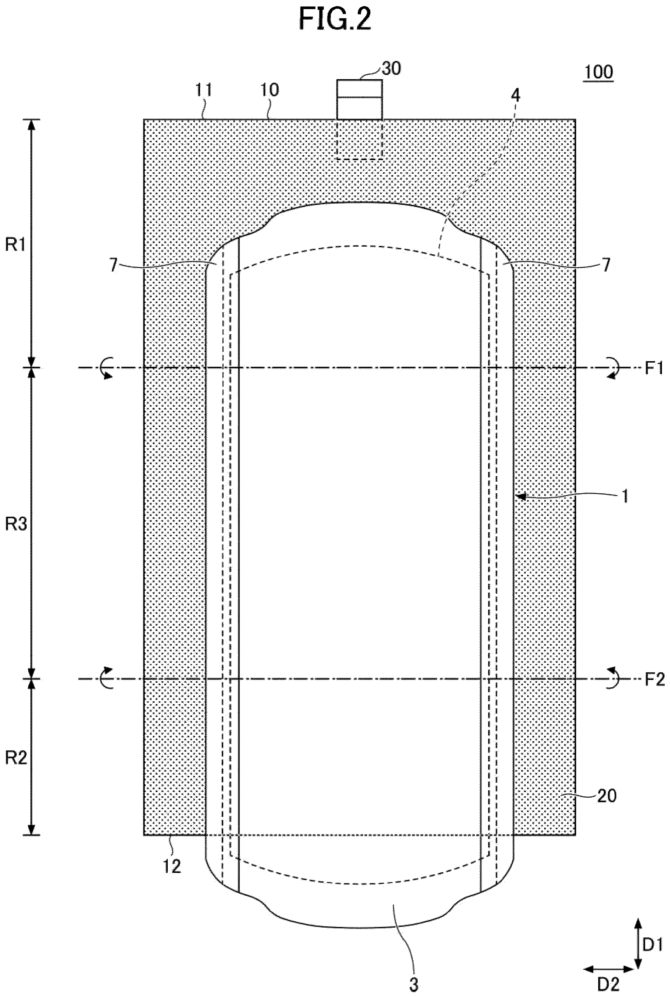

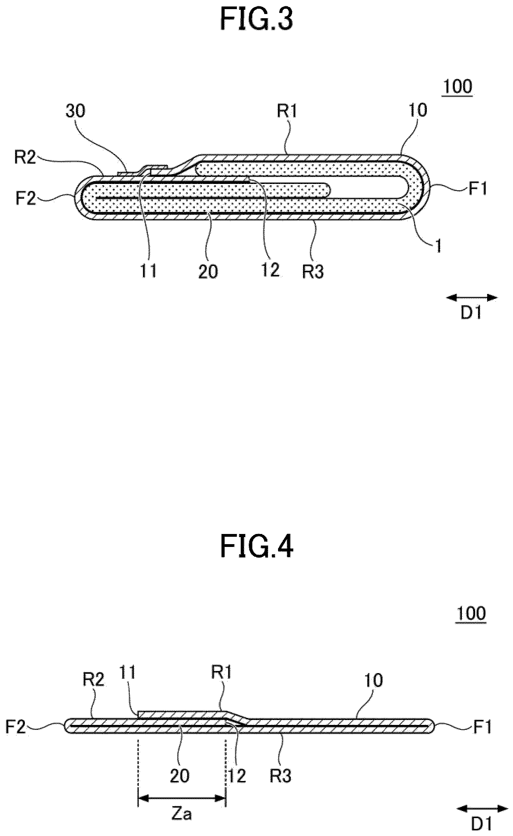

First, a basic structure of the individually packaged absorbent article will be described. FIG. 1 illustrates a plan view of an individually packaged absorbent article 100 according to the first embodiment. FIG. 2 is a plan view of a packaging sheet 10 of the individually packaged absorbent article 100 of FIG. 1 in an unfolded state, as viewed from the inner surface of the packaging sheet 10, namely from the skin side of the absorbent article 1. FIG. 3 is a sectional view taken along line I-I of FIG. 1, and FIG. 4 is a sectional view taken along line II-II of FIG. 1.

As illustrated in FIGS. 1 through 4, the individually packaged absorbent article 100 includes a packaging sheet 10 and an absorbent article 1 individually packaged by the packaging sheet 10. As illustrated in FIG. 2, the packaging sheet 10 may have an elongated shape in an unfolded state, and has a longitudinal direction (first direction) D1 and a lateral direction (second direction) D2 orthogonal to the longitudinal direction. The longitudinal direction D1 and the lateral direction D2 of the packaging sheet 10 may correspond to the longitudinal direction and the lateral direction of the absorbent article 1, respectively.

### (Absorbent Article)

The absorbent article 1 packaged by the packaging sheet 10 may be a flat and elongated article to be worn so as to face a discharge orifice of a body fluid (menstrual blood, vaginal discharge, urine, or the like). Specific examples of the absorbent article 1 may include a sanitary napkin, a panty liner, and a light incontinence pad. The description hereinafter is primarily based on the embodiment wherein the absorbent article is a sanitary napkin.

The absorbent article 1 may include, for example, as illustrated in FIG. 2, a liquid permeable top sheet 3, a liquid impermeable back sheet (not illustrated), and an absorber 4 arranged between the top sheet 3 and the back sheet.

As the back sheet, a sheet material having at least water impermeability, such as an olefin-based resin sheet of polyethylene, polypropylene, or the like, can be used. A laminated nonwoven fabric obtained by laminating a nonwoven fabric on a polyethylene sheet or the like, or a laminated sheet of a nonwoven fabric which is substantially liquid-impermeable by interposing a waterproof film therebetween may be used. Alternatively, a material having moisture permeability may be used.

As the top sheet 3, a porous or non-porous nonwoven fabric, a porous plastic sheet, or the like is preferably used. As the material fiber constituting the nonwoven fabric, for example, synthetic fibers, such as olefin such as polyethylene and polypropylene, polyester, and polyamide, regenerated fibers such as rayon and cupra, mixed fibers thereof, and natural fibers such as cotton, can be used alone or in combination of two or more kinds.

The absorber 4 is not limited as long as it is a material capable of absorbing and retaining body fluids, but preferably includes cotton-like pulp and a water-absorbent polymer. As the water-absorbent polymer, superabsorbent polymer granular powder (superabsorbent polymer (SAP)), superabsorbent polymer fiber (superabsorbent fiber (SAF)), and a combination thereof can be used. Examples of the pulp include chemical pulp obtained from wood, cellulose fibers such as dissolving pulp, and artificial cellulose fibers such as rayon and acetate. The pulp may be hardwood pulp obtained from hardwood, softwood pulp obtained from softwood, or a mixed pulp thereof. The pulp may be recycled pulp regenerated from used pulp.

The thickness of the absorber 4 may be 0.5 mm to 25 mm. The absorber 4 may have a structure in which a region facing the body fluid discharge orifice (a body fluid discharge orifice facing region) or a region facing the groove of the buttocks located behind the body fluid discharge orifice facing region is bulged. The absorber 4 has a size and shape that do not allow the absorber 4 to protrude from the top sheet 3 and the back sheet, and at the front and rear end edge portions of the absorber, the outer edges of the back sheet and the top sheet 3 are bonded by an adhesive such as hot melt or by an adhesion means such as heat sealing or ultrasonic sealing.

As illustrated in FIG. 2, in the periphery of the sides of the absorber 4, side sheets 7, 7 may be provided at respective ends of the lateral direction D2, along the longitudinal direction D1. As the side sheet 7, a water-repellent nonwoven fabric or a hydrophilic nonwoven fabric can be used.

In the example illustrated in FIGS. 1 through 4, the absorbent article 1 is a so-called wingless type, but may be configured as an article having wings extending to the sides. The wings may be formed by bonding the side sheets and the back sheet.

A displacement-prevention adhesive portion for preventing the absorbent article 1 from being displaced from underwear when the absorbent article 1 is attached to the underwear may be provided on the side of the back sheet of the absorbent article 1 (the non-skin side, namely the side facing the underwear when the absorbent article 1 is worn). The displacement-prevention adhesive portion may be formed in a plurality of bands extending in the longitudinal direction D1 or the lateral direction D2.

The total length of the absorbent article 1 can be 140 mm to 430 mm, and the breadth of the absorbent article 1 (the breadth of the main body excluding the wings in the case of having wings) can be 40 mm to 130 mm.

### (Packaging Structure)

As illustrated in FIGS. 1 through 4, the absorbent article 1 is packaged by being folded together with the packaging sheet 10, and an individually packaged absorbent article 100 is formed. When being folded, as illustrated in FIG. 2, the absorbent article 1 is placed on the inner surface of the packaging sheet 10 so that the back sheet side of the absorbent article 1 faces the inner surface of the packaging sheet 10. Then, the packaging sheet 10 and the absorbent article 1 are folded together toward the inner surface side in the longitudinal direction D1 along a first fold line F1 and a second fold line F2 along the width direction D2. More specifically, a first region R1 including one end 11 of the packaging sheet 10 in the longitudinal direction D1 is folded back in the longitudinal direction D1 along the first fold line F1, and a second region R2 including the other end 12 of the packaging sheet 10 in the longitudinal direction D1 is folded back along the second fold line F2. The region between the first region R1 and the second region R2 of the packaging sheet 10 is a third region R3. In the illustrated example, the packaging sheet 10 is folded in such a manner that the second region R2 is folded first and the first region R1 is folded to overlap the outer surface of the second region R2 (FIGS. 1 and 2), but the order of folding the first region R1 and the second region R2 may be reversed. In this way, after the absorbent article 1 is packaged by the packaging sheet 10 which is inwardly folded into three sections together with the absorbent article 1, both edge portions in the lateral direction D2 are sealed along the longitudinal direction D1, and sealing portions 15, 15 are formed.

Such a packaging folded into three or more sections can be formed relatively simply, and it is possible to package the absorbent article 1 hygienically and to easily take out the absorbent article 1, with such a packaging. The way of folding is not limited to folding in three, and may be folding in four or more (described later in detail), or may be folding in two.

As illustrated in FIG. 1, the sealing portions 15, 15 may be formed over the entire length of the packaging sheet 10 in the longitudinal direction D1. The sealing portions 15 and 15 can prevent dust, dirt, a finger, or a small object from entering the individually packaged absorbent article 100 from the end edge in the lateral direction D2 (sealing properties can be obtained). The range of the lateral direction D2 in which the sealing portion 15 is provided may be 20 mm from the end edge in the lateral direction D2. The sealing portion 15 may be formed in the entire range or may be formed in a part of the range, for example, at a position away from the end edge of the lateral direction D2. A length (width) of the sealing portion 15 in the lateral direction D2 may be 3 mm to 15 mm.

The sealing portions 15, 15 may be formed by, for example, inserting both edge portions of the folded packaging sheet 10 (both edge portions in the lateral direction D2 of the packaging sheet 10) through a pair of rolls, and pressing and heating the edge portions with the rolls to press layers of the folded packaging sheet 10 against each other in the thickness-wise direction. The pair of rolls may be, for example, a combination of a roll having a plurality of protrusions on the surface and a roll having no irregularities on the surface. In this case, the plurality of protrusions of one roll are pressed against the packaging sheet, and a plurality of pressed portions corresponding to the protrusions can be formed on the packaging sheet. Alternatively, the surfaces of both rolls may be formed with irregularities that engage with each other, and the packaging sheet may be deformed by the engagement. When the sealing portion is formed, the rolls may be heated or need not be heated. In the case where a packaging sheet coated with a heat sealing agent, which will be described in detail later, is used, heat sealing can be formed by heating and pressurizing.

### (Material of Packaging Sheet)

The packaging sheet 10 in the present embodiment is made of paper. The use of the paper packaging sheet 10 can contribute to reduction of plastics and achievement of sustainable development goals. Paper can also provide comfort to users because it can provide a unique texture, such as a gentle look and feel of a natural material. In this specification, "paper" refers to a thin flat sheet obtained by agglutinating plant fibers or other fibers with an agglutinating agent. In particular, "paper" refers to a sheet containing a plant fiber as a main raw material, for example, one containing 50% or more, preferably 80% or more, of a plant fiber, particularly a cellulose fiber, among the contained fibers. Examples of the plant fiber as a raw material of paper include wood pulp, non-wood pulp, waste paper pulp, and cotton cellulose, and the pulp may be any of mechanical pulp and chemical pulp. The plant fibers may be regenerated fibers such as rayon and cupra, or may be plant fibers recycled from a constituent element of an absorbent article and/or a packaging sheet for an absorbent article (recycled pulp or the like).

The paper may contain known paper additives, such as clay, calcium carbonate, starch, latex, color pigments, preservatives, and the like. Specific examples of the paper include various types of paper such as Western paper, Japanese paper, processed paper, and synthetic paper. Further, the paper may be paper conventionally used for other purposes, for example, paper called newsprint paper, printing paper (including high-quality paper), writing paper, drawing paper, packaging paper, tissue paper, hybrid paper, or the like. As thin paper, thin vellum paper, Indian paper, rice paper, glassine paper, tissue paper, toilet paper, filter paper, or the like may be used.

A basis weight of the packaging sheet 10 (a basis weight before a strength improving layer is provided) used in the present embodiment may be preferably 8 g/m² to 50 g/m², more preferably 10 g/m² to 30 g/m² or less. By using the packaging sheet 10 having a basis weight in the above range, an individually packaged absorbent article having a good texture with a suppressed stiff feel can be obtained, and the generation of noise during carrying, unsealing, and the like can be prevented. The thickness of the packaging sheet 10 (the thickness after creping in the case of crepe paper) may be preferably 30 µm to 200 µm, and more preferably 35 µm to 100 µm.

The dimensions of the packaging sheet 10 may be determined according to the size and shape of the absorbent article 1 to be packaged. For example, the dimension of the longitudinal direction D1 (sometimes simply referred to as a "length") may be 100 mm to 450 mm, and the dimension of the lateral direction D2 (sometimes simply referred to as a "width") may be 70 mm to 250 mm, when the packaging sheet 10 is completely unfolded (not folded). In the example illustrated in the drawings, the packaging sheet 10 has a rectangular shape in an unfolded state, but may have a shape such as an oblong shape.

In the individually packaged absorbent article 100, a fastening tape 30 may be provided at the center of the width direction D2 so as to straddle the one end 11 of the packaging sheet 10, that is, so as to straddle the first region R1 and the second region R2 on the outer surface of the individually packaged absorbent article 100. The fastening tape 30 can prevent an object, a finger, or the like from entering and being caught between the first region R1 and the second region R2, and prevent the seal from being unintentionally unsealed before use. Further, since the first region R1 can be lifted up by holding the fastening tape 30 at the time of unsealing, an act of unsealing is facilitated.

### (Strength Improving Layer)

The packaging sheet 10 in the present embodiment is provided with a strength improving layer on the inner surface and/or the outer surface. The strength improving layer may be a layer that improves one or more of the tensile strength, tear strength, and burst strength of the paper packaging sheet 10, and preferably may be a layer that improves the tensile strength (the tensile strength in at least one of the MD (machine direction) and the CD (cross direction) of the paper). The tensile strength can be measured by using, for example, a known tensile tester. In the examples illustrated in FIGS. 2 through 4, the strength improving layer 20 is provided on the inner surface of the paper packaging sheet 10, but the strength improving layer 20 may be provided on the outer surface, or may be provided on both the inner surface and the outer surface. The strength of the packaging sheet 10 can be improved regardless of whether the strength improving layer 20 is provided on the inner surface or the outer surface. In the case where the reinforcing member is provided on both the inner surface and the outer surface, the effect of improving the strength is further enhanced.

By providing the strength improving layer, the strength of the packaging sheet can be improved. Therefore, even when a large force is locally applied to the packaging sheet at the time of unsealing a package, at the time of housing the individually packaged absorbent article in a package during manufacturing, at the time of carrying the individually packaged absorbent article before starting use, or the like, the packaging sheet can be prevented from being damaged. Therefore, the packaging sheet can be smoothly unfolded without being torn during unsealing. When an absorbent article is replaced, the used absorbent article can be wrapped with a packaging sheet that is not damaged or hardly damaged.

The strength improving layer 20 can be formed by performing a strength improving layer forming process, for example, by applying a strength improving agent to the packaging sheet 10. Further, a layer or sheet having a function of improving strength may be bonded to the packaging sheet 10 to form a laminated sheet.

In the case where the strength improving layer 20 is formed by applying the strength improving agent, the strength improving agent containing a resin is preferable. When such a strength improving agent is applied, it can be used in a liquid form, for example, in the form of a liquid resin, a resin solution, or a resin dispersion. As the strength improving agent, a heat sealing agent, an anti-slipping agent, a filler, and the like can be used. Thus, the strength improving agent having a function of improving the strength of the packaging sheet 10 and a function additional to this function is preferable.

The surface on which the strength improving layer 20 is provided can be appropriately selected depending on the type of the strength improving layer 20. More specifically, the strength improving layer 20 can be selected according to its own additional function (a function other than the strength improving function of the packaging sheet 10). For example, in the case where it is desired to apply a barrier for preventing bleeding-through of ink on the outer surface of the packaging sheet 10, a barrier agent may be selected as the strength improving layer 20 and the barrier agent may be applied to the outer surface of the packaging sheet 10. This improves the strength of the packaging sheet 10 and also exhibits a barrier function.

In the case where the strength improving agent is applied, printing such as gravure printing, flexographic printing, offset printing, or screen printing, application with a spray gun, a dispenser, or a roll coater, or the like can be used. Among these, flexographic printing is preferably used because it enables mass printing using an inexpensive aqueous ink and thus can reduce the cost.

In the case where the strength improving layer 20 is formed by lamination, a known lamination technique can be used. In this case, the layer or sheet having a function of improving strength, which is prepared in advance, may be a resin film made of an olefin resin such as polyester or polypropylene, or a resin film made of polyvinyl chloride or the like.

In the case where the strength improving layer 20 is formed by applying a strength improving agent or by laminating a layer or sheet having a strength improving function, the strength of the packaging sheet after the formation of the strength improving layer is preferably increased by 10% or more with respect to the strength of the packaging sheet before the formation of the strength improving layer. In addition, in the case where the strength improving layer 20 is formed by laminating layers or sheets, the thickness of the layer or sheet having a strength improving function may preferably be more than 0 µm to 20 µm, and more preferably more than 0 µm to 10 µm.

Even in the case where the strength improving layer 20 is formed on either the inner surface or the outer surface of the packaging sheet 10, the formation range of the strength improving layer 20 may be the entire surface of the packaging sheet 10 or a part of the surface. In the example illustrated in FIG. 2, the strength improving layer 20 is formed on the entire inner surface of the packaging sheet 10. In the case where the strength improving layer 20 is formed on the entire surface, the strength can be improved over the entire packaging sheet 10. Not separating the region where the strength improving layer 20 is formed and the region where the strength improving layer 20 is not formed from each other is preferable from the viewpoint of not making the process of forming the strength improving layer 20 complicated. In the region where the strength improving layer 20 is formed, that is, in the region to which the process of forming the strength improving layer 20 is applied, the strength improving layer 20 may be formed in a solid manner (continuously without a gap in the plane direction) or may be formed in a discontinuous pattern. In the latter case, the strength improving layer 20 may be formed in a discontinuous pattern that can be recognized by a user by viewing it by a normal method, or may be formed in an arrangement that cannot be recognized by a user by viewing it by a normal method or is difficult to recognize by a user, for example, in a manner that the strength improving layer 20 is microscopically scattered in a size-varying dots pattern.

The paper packaging sheet 10 may be subjected to any processing other than the formation of the strength improving layer 20 as long as the function of the strength improving layer 20 is not hindered. This processing may include processing for applying physical deformation to the packaging sheet 10, such as creping, embossing, calendering, slitting, and plying.

In addition to the formation of the strength improving layer 20, ink may be printed on the outer surface of the packaging sheet 10. By performing ink printing, the design of the individually packaged absorbent article is improved, and the packaging sheet 10 can be reinforced from the outer surface. In the case where the ink printing is performed on the packaging sheet 10, it is preferable to use a material (uncreped paper or unembossed paper) that is not subjected to a process of forming irregularities on the surface of the packaging sheet, such as creping or embossing, as the packaging sheet 10.

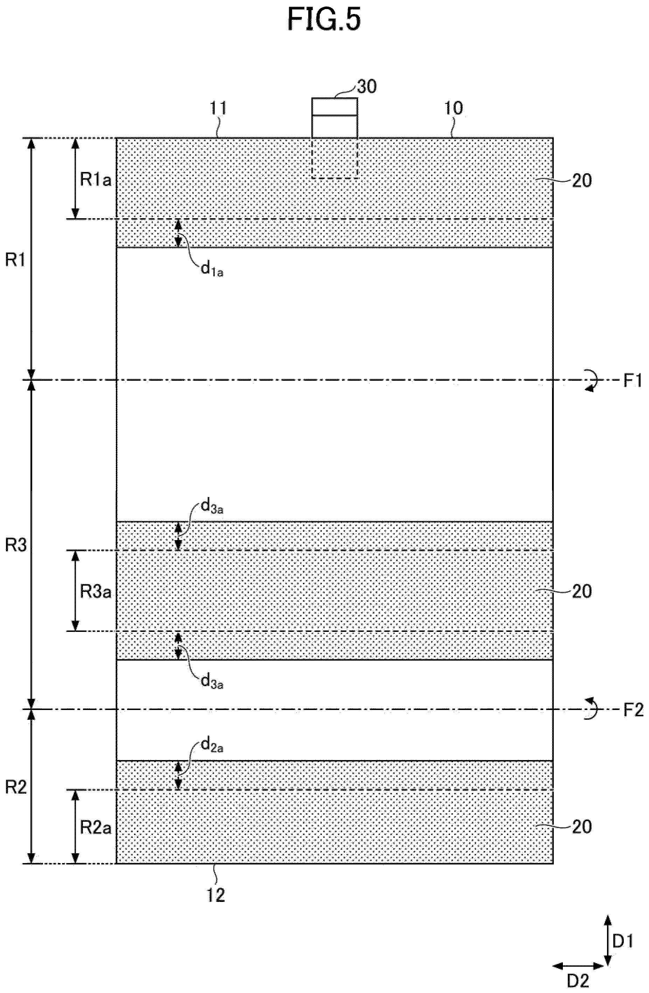

FIG. 5 illustrates a modified example of the strength improving layer 20 provided on the packaging sheet 10. In the example illustrated in FIG. 5, the strength improving layer 20 is provided not on the entire packaging sheet 10 but on a part of the packaging sheet 10.

Referring again to FIGS. 1 and 4, in the individually packaged absorbent article 100 having a tri-fold packaging structure as in the present embodiment, the number of layers of the packaging sheet 10 varies depending on the location. For example, as illustrated in FIG. 1, the location where the first region R1 and the second region R2 overlap is a thick zone Za where the packaging sheet 10 is layered to have three layers. The thick zone Za is also referred to as a "three-or-more-ply zone". In the formation of the sealing portions 15, 15 using the pair of rolls described above, the axes of the rolls are usually arranged in a direction along the longitudinal direction D1, and therefore, the seal is formed at the same timing along the longitudinal direction D1 and with the same strength along the longitudinal direction. Therefore, in the thick zone Za, the degree of compression of the layered packaging sheet 10 is larger than in other zones where the number of layers is smaller, and therefore the sealing strength of the sealing portions 15, 15 tends to be stronger. Herein, when the seal is peeled off at the time of unsealing, in the zone where the sealing strength is high, the sealing strength is higher than the strength of the packaging sheet, and the possibility that the packaging sheet would be damaged increases. Therefore, by reinforcing the region of the packaging sheet 10 included in the thick zone Za, it is possible to effectively prevent the packaging sheet 10 from being damaged when the packaging sheet 10 is unsealed.

Therefore, for example, as in the modified example illustrated in FIG. 5, the strength improving layer 20 can be formed at least in any of the regions corresponding to the thick zone Za (FIGS. 1 and 4) where the packaging sheet is layered to have three layers. More specifically, the strength improving layer 20 can be formed in one or more of the region R1a of the first region R1 that overlaps the second region R2 in the state of the individually packaged absorbent article 100, the region R2a of the second region R2 that overlaps the first region R1 in the state of the individually packaged absorbent article 100, and the region R3a of the third region R3 that corresponds to the portion where the first region R1 and the second region R2 overlap each other. It is preferable that the strength improving layer 20 is formed in all of the region R1a, the region R2a, and the region R3a.

Further, as illustrated in FIG. 5, the strength improving layer 20 may be provided in one or more of a region including the region R1a, a region including the region R2a, or a region including the region R3a. For example, the region R1a is a region starting from one end 11 of the packaging sheet 10 in the longitudinal direction D1, and the strength improving layer 20 extends to a position beyond the position of the end of the region R1a in the longitudinal direction D1. The region R2a is a region starting from one end 12 of the packaging sheet 10 in the longitudinal direction D1, and the strength improving layer 20 extends to a position beyond the position of the end of the region R2a in the longitudinal direction D1. The strength improving layer 20 extends beyond the positions of both ends of the region R3a in the longitudinal direction D1 in the third region R3. Thus, the strength improving layer 20 is formed not only in the region (region R1a, region R2a, region R3a) corresponding to the thick zone Za of the packaging sheet 10, but also in a region larger than the region so as to include the region, whereby the region of the packaging sheet 10 corresponding to the portion adjacent to the thick zone Za is also reinforced, which is preferable.

In the case where the strength improving layer 20 extends beyond the region corresponding to the thick zone Za, the extension length of the longitudinal direction D1, in particular, the extension length d1a extending beyond the end of the region R1a in the longitudinal direction D1, the extension length d2a extending beyond the end of the region R2a in the longitudinal direction D1, and the extension length d3a extending beyond both ends of the region R3a in the longitudinal direction D1 (FIG. 5) may be more than 0 mm to 20 mm, or 5 mm to 10 mm. The extension length d3a is present on each of the sides of the longitudinal direction D1, and the lengths may be the same or different.

In the example as illustrated in FIG. 5, the strength improving layer 20 extends across the lateral direction D2, i.e., from one edge to the other edge in the lateral direction D2. This configuration is preferable because, in the case where the strength improving agent is applied with the lateral direction D2 as the conveyance direction, there is no need to switch between application and no application of the agent during conveyance of the packaging sheet. In the present example, since there is a region where the strength improving layer 20 is not provided in the packaging sheet 10, the strength of the packaging sheet 10 can be effectively improved while the material of the strength improving layer 20 is reduced due to the absence of the strength improving layer 20.

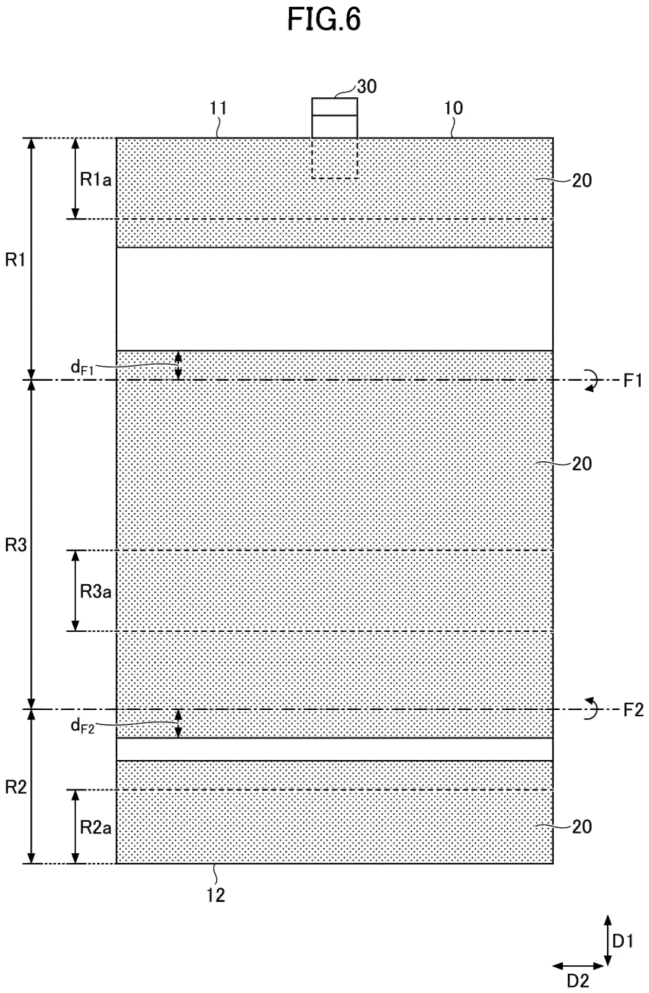

FIG. 6 illustrates another modified example of the arrangement of the strength improving layer 20. In the example illustrated in FIG. 6, the strength improving layer 20 is formed in each of the region including the region R1a, the region including the region R2a, and the region including the region R3a, as in the example illustrated in FIG. 5. However, the region including the region R3a where the strength improving layer 20 is formed is longer in the longitudinal direction D1 than in the example illustrated in FIG. 5. In the example illustrated in FIG. 6, the strength improving layer 20 at the center in the longitudinal direction D1 is formed so as to include the third region R3, more specifically, so as to extend beyond the first fold line F1 and the second fold line F2.

The first fold line F1 and the second fold line F2 are portions that are easily damaged by being hit by a device, a human hand, or the like in the case where the individually packaged absorbent article 100 is accommodated in a package or carried; therefore, the packaging sheet 10 that is hardly damaged is obtained by reinforcing the packaging sheet 10 at the position of the first fold line F1 and/or the second fold line F2. In the example in FIG. 6, since there is a region where the strength improving layer 20 is not provided in the packaging sheet 10, the strength of the packaging sheet 10 can be effectively improved while the material of the strength improving layer 20 is reduced.

In FIG. 6, the extension length dF1 of the strength improving layer 20 extending beyond the first fold line F1 and the extension length dF2 of the strength improving layer 20 extending beyond the second fold line F2 may each be in a range of more than 0 mm to 10 mm.

### <Second Embodiment>

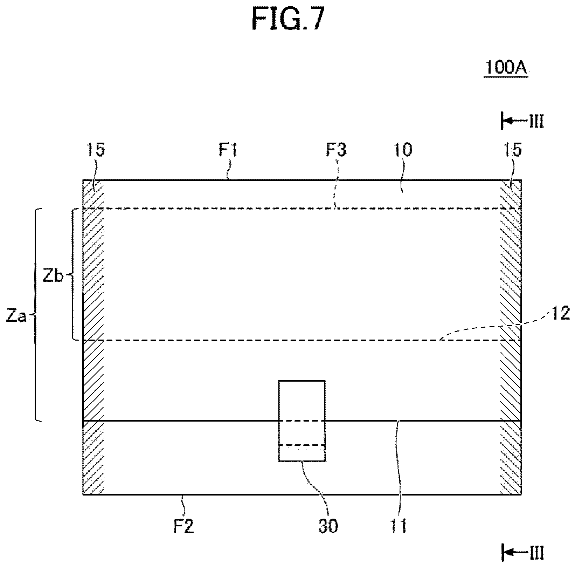

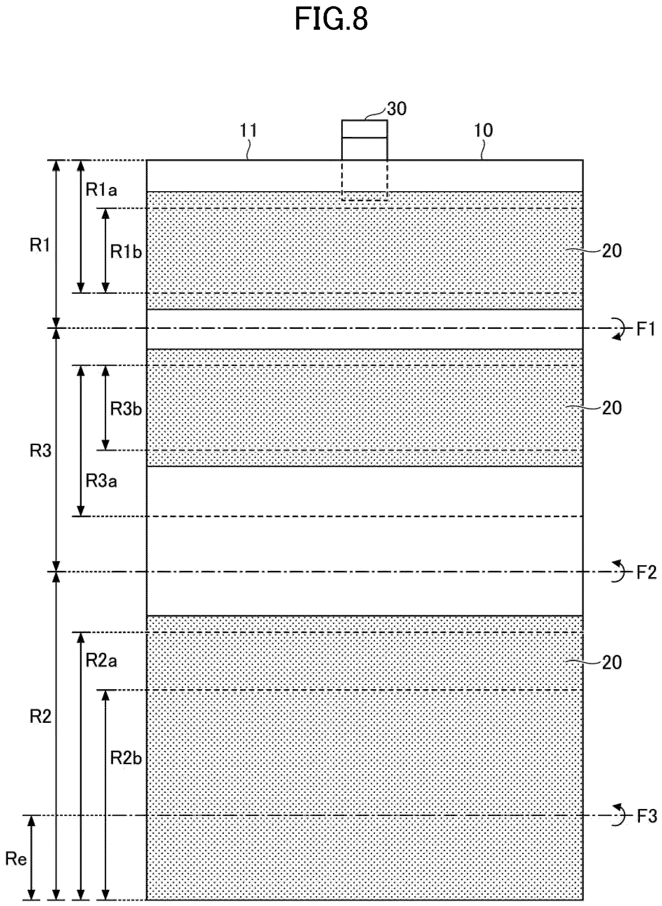

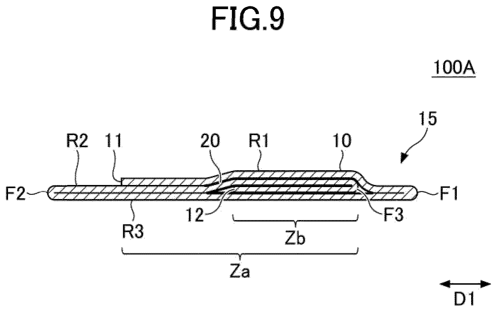

FIG. 7 illustrates a plan view of the individually packaged absorbent article 100A according to the second embodiment, and FIG. 8 illustrates a plan view of the individually packaged absorbent article 100A of FIG. 7 in a state where the packaging sheet 10 is unfolded, as viewed from the inner surface, but the absorbent article 1 is not illustrated in FIG. 7 and FIG. 8. FIG. 9 is a cross-sectional view taken along line III-III of FIG. 7. Although the following second embodiment will be described based on an example in which the strength improving layer 20 is provided on the inner surface of the packaging sheet 10, the strength improving layer 20 may be formed on the outer surface of the packaging sheet 10 or on both surfaces of the packaging sheet 10, depending on the type of the strength improving layer 20 as described above.

As in the first embodiment (FIGS. 1 through 6), the individually packaged absorbent article 100A according to the second embodiment also has a packaging configuration in which an absorbent article, such as a sanitary napkin, is arranged on the inner surface of a packaging sheet and both are folded together, but in the individually packaged absorbent article 100A according to the present example, an absorbent article having a length in the longitudinal direction D1 longer than that in the first embodiment is packaged, and the packaging sheet 10 is folded into four sections together with the absorbent article.

In the four-fold packaging structure of the present embodiment, the basic way of folding is the same as that for the three-fold packaging structure, and the packaging sheet 10 is folded to the inner surface side so that the first region R1 having one end 11 of the packaging sheet 10 in the longitudinal direction D1 and the second region R2 having the other end 12 overlap each other. However, before the second region R2 is folded, the end region Re including the other end 12 is folded back along a third fold line F3 located between the second fold line F2 and the other end 12. In the example illustrated in FIG. 8, the end region Re is folded back to the inner surface side of the packaging sheet 10, but may be folded back to the outer surface side. In the individually packaged absorbent article 100 obtained after the packaging sheet 10 is folded into four sections, a zone in which the packaging sheet is layered to have two layers, a zone in which the packaging sheet is layered to have three layers, and a zone in which the packaging sheet is layered to have four layers are formed. In other words, as illustrated in FIGS. 7 and 9, a zone where the packaging sheet is layered to have three layers ("three-or-more-ply zone" or "thick zone Za") is formed at the center of the longitudinal direction D1, and a four-ply zone Zb is formed in the three-or-more-ply zone Za. Depending on the packaging structure, the four-ply zone Zb may include a zone where the packaging sheet 10 is layered to have five or more layers.

As described above, the sealing strength of the seals 15, 15 tends to be stronger in a zone where the number of layers of the packaging sheet is larger, and the seals 15, 15 are more likely to be damaged when the packaging sheet 10 is unsealed. Therefore, it is preferable to reinforce the region of the packaging sheet 10 included in the three-or-more-ply zone Za. In other words, it is preferable that the strength improving layer 20 is formed in one or more of the region R1a of the first region R1 overlapping the second region R2, the region R2a of the second region R2 overlapping the first region R1, and the region R3a of the third region R3 corresponding to the portion where the first region R1 and the second region R2 overlap each other. It is preferable that the strength improving layer 20 is formed in all of the region R1a, the region R2a, and the region R3a.

In the four-ply structure of the present embodiment, as illustrated in FIG. 8, it is preferable to reinforce at least the region of the packaging sheet 10 included in the four-ply zone Zb. Therefore, it is preferable that the strength improving layer 20 is formed in one or more of the region R1b included in the four-ply zone Zb of the packaging sheet in the first region R1, the region R2b included in the four-ply zone Zb of the packaging sheet in the second region R2, and the region R3b included in the four-ply zone Zb of the packaging sheet in the third region R3.

As described with reference to FIG. 5, the strength improving layer 20 provided in any of the region R1a, the region R2a, and the region R3a may be formed so as to include the region R1a, the region R2a, and the region R3a, respectively, in other words, so as to extend beyond the end edges of the region R1a, the region R2a, and the region R3a in the longitudinal direction D1, respectively. As illustrated in FIG. 8, the strength improving layer 20 may extend beyond positions of both ends of the region R1b in the longitudinal direction D1, beyond the position of an end of the region R2b in the longitudinal direction D1, and beyond positions of both ends of the region R3b in the longitudinal direction D1. The extension length of each of the region R1b, the region R2b, and the region R3b extending beyond the end positions in the longitudinal D1 of these regions may be more than 0 mm to 20 mm, or 5 mm to 10 mm. However, the strength improving layer 20 formed in the range including the region R1b may overlap the fastening tape 30 in a plan view as illustrated in FIG. 8. Since the peripheral portion of the packaging sheet 10 where the fastening tape 30 is provided is also a portion where force is likely to be concentrated at the time of unsealing and thus damage is likely to occur, as illustrated in FIG. 9, the portion in the vicinity of where the fastening tape 30 is provided is reinforced by the strength improving layer 20 overlapping the fastening tape 30 in a plan view.

In the second embodiment, the strength improving layer 20 is formed not on the entire surface of the packaging sheet 10 but in the region where damage to the packaging sheet 10 easily occurs at the time of unsealing, and the strength improving layer 20 is not formed in the region where damage to the packaging sheet 10 hardly occurs, whereby the strength of the packaging sheet 10 can be efficiently improved while the material of the strength improving layer 20 is reduced. From the viewpoint of improving the strength of the entire packaging sheet 10, it is preferable that the strength improving layer 20 is provided over the entire surface of the packaging sheet 10 as in the example illustrated in FIG. 2.

### <Third Embodiment>

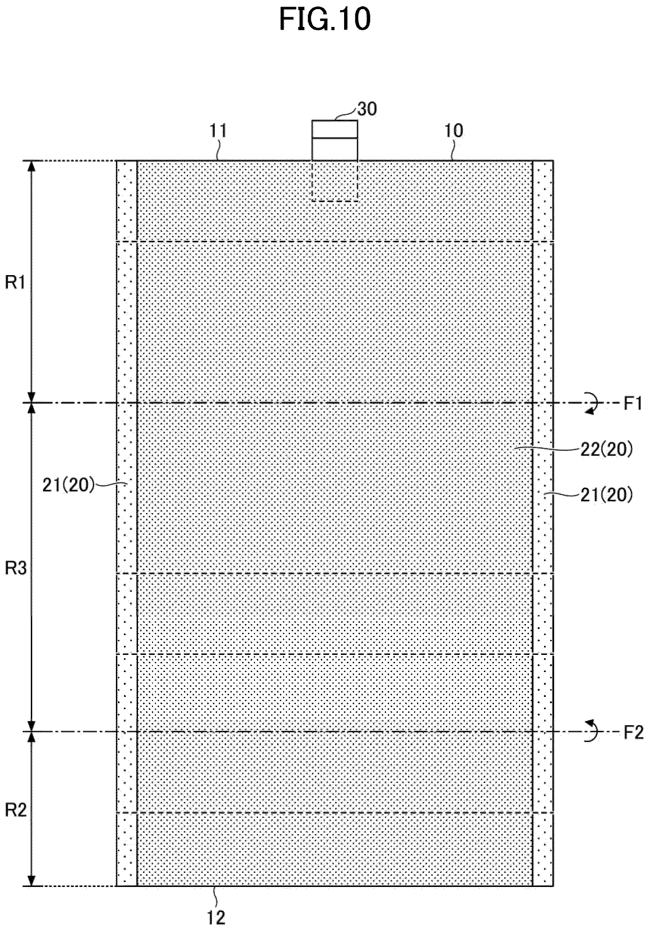

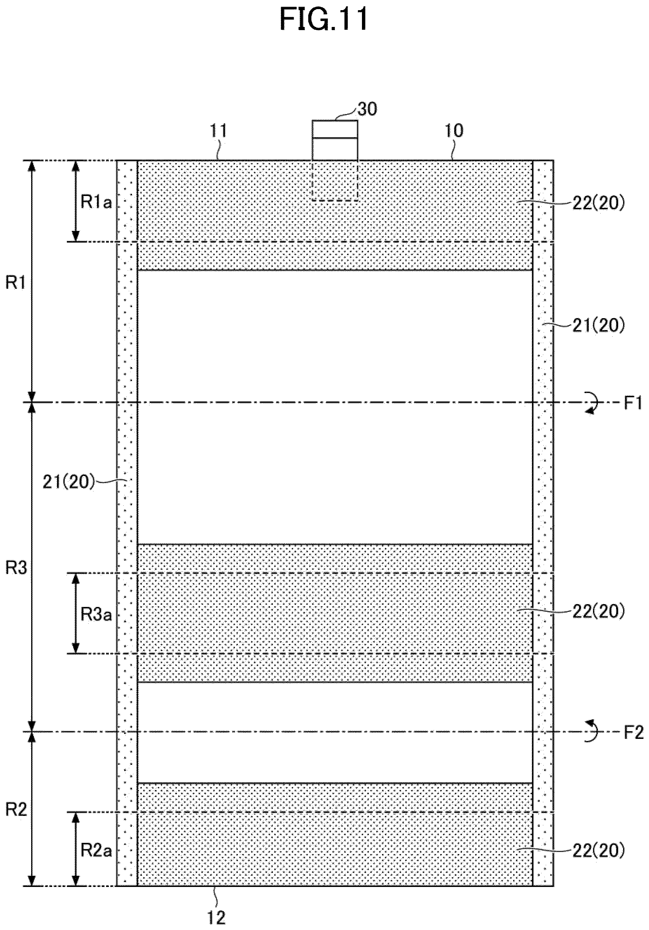

FIGS. 10 and 11 are plan views illustrating the inner surface of the packaging sheet 10 of the individually packaged absorbent article according to the third embodiment in an unfolded state. Although the absorbent article 1 is not illustrated in FIGS. 10 and 11, the structure for packaging the absorbent article 1 is the same as that of the first embodiment (FIGS. 1 through 4, etc.) also in the embodiment illustrated in FIGS. 10 and 11. Although the following third embodiment will be described based on an example in which the strength improving layer 20 is provided on the inner surface of the packaging sheet 10, the strength improving layer 20 may be formed on the outer surface of the packaging sheet 10 or on both surfaces of the packaging sheet 10, depending on the type of the strength improving layer 20 as described above.

In the third embodiment, the strength improving layer 20 is formed by applying a heat sealing agent. The heat sealing agent is an agent that is interposed between two materials to thereby assist the bonding of the two materials. The heat sealing agent may contain a thermoplastic resin, and is preferably an olefin-based heat sealing agent. The heat sealing agent may be a water dispersion of resin particles. Specific examples of the heat sealing agent include "Chemipearl (registered trademark)" series manufactured by Mitsui Chemicals, Inc.

When the strength improving agent is a heat sealing agent, heat sealing can be used for forming the sealing portions 15, 15 (FIG. 1) of the paper packaging sheet 10. The sealing strength of the sealing portion formed by heat sealing can be higher than that of a seal formed by physical deformation of the packaging sheet without an intervention of an agent.

In this embodiment, the heat sealing agent may be applied to the entire surface of the packaging sheet 10 as illustrated in FIG. 2, or may be applied to only a part of the surface of the packaging sheet 10. However, in the case where the heat sealing agent is uniformly applied to the entire inner surface, the sealing strength of the sealing portions 15, 15 may be excessively increased depending on the type of the heat sealing agent and the conditions during heat sealing. Therefore, the basis weight of the strength improving layer 20 can be made different between the edge portions of the packaging sheet 10 in the lateral direction D2 where the sealing portion 15 is formed and the other region. For example, as illustrated in FIG. 10, the basis weight of the edge strength improving layers 21, 21 formed on both edges of the lateral direction D2 can be made smaller than the basis weight of the central portion strength improving layer 22 in the region between the edge strength improving layers 21, 21.

On the other hand, depending on the type of the heat sealing agent and the conditions at the time of heat sealing, in the case where the heat sealing agent is uniformly applied to the entire surface, the sealing strength may be weakened although the packaging sheet 10 is sufficiently reinforced. In such a case, the basis weight of the edge strength improving layers 21, 21 may be made larger than the basis weight of the central portion strength improving layer 22 in the region between the edge strength improving layers 21, 21.

The central portion strength improving layer 22 need not be formed continuously over the longitudinal direction D1, and may be formed discontinuously in the longitudinal direction D1 as illustrated in FIG. 11. The formation range of the central portion strength improving layer 22 in FIG. 11 corresponds to the formation range of the strength improving layer 22 described with reference to FIG. 5. In other words, the central portion strength improving layer 22 is formed in a region including the region R1a included in the thick zone (three-or-more-ply zone) Za (FIGS. 1 and 4), a region including the region R2a, and a region including the region R3a. In the example illustrated in FIG. 11, the strength of the region corresponding to the thick zone (three-or-more-ply zone) Za where damage is likely to occur can be improved in the main region between both edge portions of the packaging sheet 10 in the lateral direction D2. Since the main region includes the region where the strength improving layer is not provided, the strength of the packaging sheet 10 can be efficiently improved while the material of the strength improving layer is reduced.

### <Fourth Embodiment>

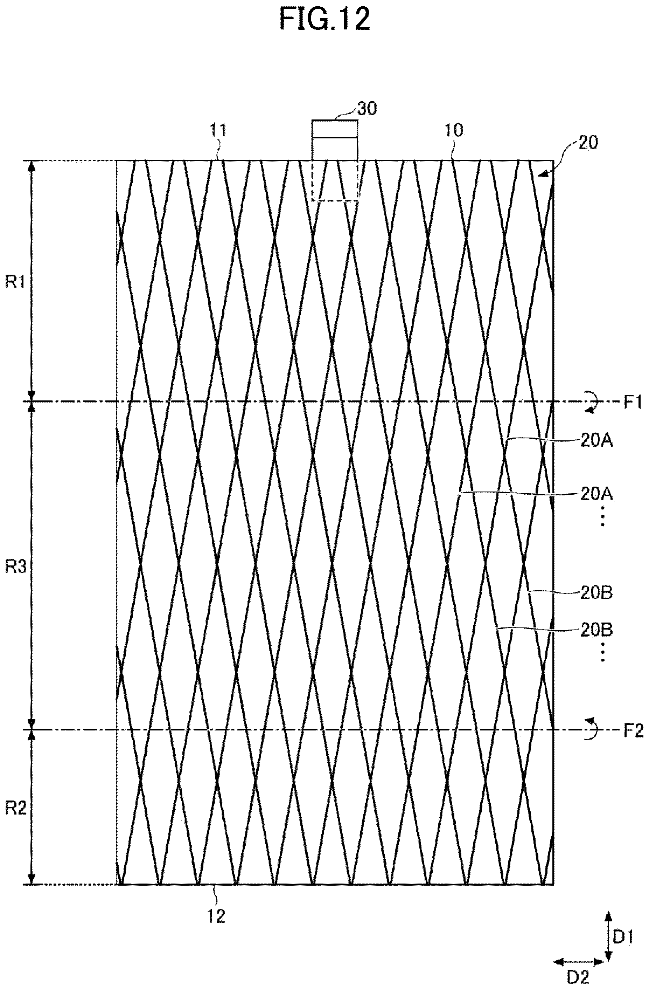

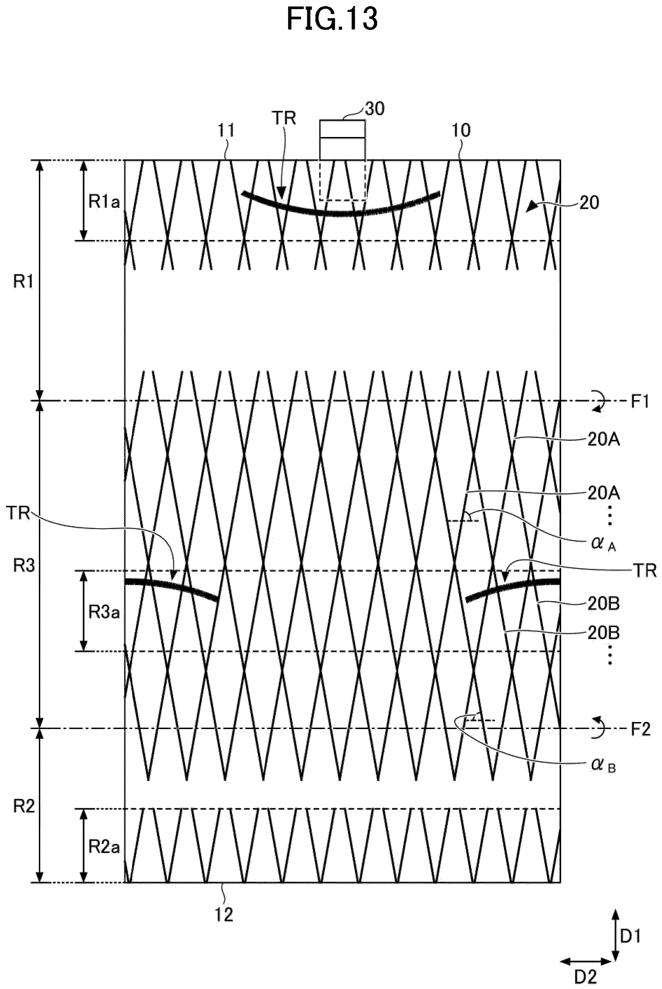

FIGS. 12 and 13 are plan views illustrating the inner surface of the packaging sheet 10 of the individually packaged absorbent article according to the fourth embodiment in an unfolded state. The fourth embodiment also has a configuration in which the same absorbent article 1 as that described in the first embodiment (FIGS. 1 through 4 and the like) is packaged with the packaging sheet 10, but the absorbent article 1 is not illustrated in FIGS. 12 and 13. Although the following fourth embodiment will be described based on an example in which the strength improving layer 20 is provided on the inner surface of the packaging sheet 10, the strength improving layer 20 may be formed on the outer surface of the packaging sheet 10 or on both surfaces of the packaging sheet 10 depending on the type of the strength improving layer 20 as described above.

In the fourth embodiment, the strength improving layer 20 is formed in a pattern. In the pattern of the strength improving layer 20 in the example illustrated in FIGS. 12 and 13, when a user views the surface of the packaging sheet 10 by a normal method, the user can recognize portions where the strength improving layer 20 is not provided within the region where the strength improving layer pattern is formed. However, the pattern of the strength improving layer 20 may be a fine pattern that is not recognizable by a user by a normal method. The pattern of the strength improving layer 20 may be, for example, a pattern including a plurality of linear portions as illustrated in FIG. 12. In the example illustrated in FIG. 12, the pattern of the strength improving layer 20 includes a plurality of linear portions 20A, 20A, ... extending along a predetermined direction and spaced apart from each other, and linear portions 20B, 20B, ... extending in a direction intersecting the plurality of linear portions 20A, 20A, ... and spaced apart from each other.

With the patterned strength improving layer 20, the strength of the packaging sheet 10 can be improved, and the original bendability, texture, and the like of paper can be more strongly maintained. Therefore, for example, in the folding process of the packaging sheet 10 at the time of manufacturing the individually packaged absorbent article, there is an advantage that the packaging sheet 10 can be easily folded. Furthermore, the material of the strength improving layer 20 can be reduced, which leads to cost reduction.

In the case where the strength improving layer 20 is formed in a pattern, the ratio of the area where the strength improving layer is disposed to the area of the region where the strength improving layer pattern is formed can be set to 40% to 95%.

The pattern of the strength improving layer 20 is not limited to that of FIG. 12, and may be a combination of linear portions extending at another angle, for example, a lattice shape, or a dot shape that can be formed by a user in a normal manner. The strength improving layer 20 may be provided in a pattern including information such as pictures and characters, or may be arranged at random. It is preferable that the pattern of the strength improving layer 20 be formed so as to prevent the packaging sheet 10 from being torn. Herein, since fibers are oriented in the traveling direction during paper making in paper which is an aggregate of fibers, paper has the MD (machine direction), which is an extending direction of the fibers (main orientation of the fibers), and the CD (cross direction) which is a direction orthogonal to the MD. Therefore, the packaging sheet 10 made of paper also has the MD and the CD, and in this case, either the longitudinal direction D1 or the lateral direction D2 may be the MD of the paper (the extending direction of the fibers of the paper), and the lateral direction D2 is often the MD. Since paper is easily torn along the MD, the linear portions of the pattern of the strength improving layer 20 are also preferably formed in a direction intersecting the MD, for example, so as to prevent tearing of the packaging sheet 10 along the MD. In other words, by forming the strength improving layer 20 in a pattern including a linear portion extending in a direction intersecting the MD of the paper, the packaging sheet 10 can be particularly effectively prevented from being torn. The relationship between the pattern of the linear portions of the strength improving layer 20 and the fiber extending direction of the packaging sheet 10 will be further described in the following modified example (FIG. 13).

FIG. 13 illustrates a modified example of the strength improving layer 20 in the fourth embodiment. The strength improving layer may be formed on a part of the inner surface as illustrated in FIG. 13, instead of being formed on the entire surface as illustrated in FIG. 12. In the case where the strength improving layer 20 is formed on a part of the surface of the packaging sheet 10, it is preferable to, as described with reference to FIG. 5, perform the strength improving layer forming process on one or more of the region R1a, the region R2a, and the region R3a corresponding to the thick zone (three-or-more-ply zone) Za where damage is likely to occur.

In the case where the strength improving layer forming process is performed on a part of the surface, it is preferable to perform the strength improving layer forming process on one or more of a region including the region R1a, a region including the region R2a, and a region including the region R3a, the regions R1a, R2a, and R3a corresponding to the thick zone (three-or-more-ply zone) Za. As described with reference to FIG. 6, it is preferable that the region including the region R3a where the strength improving layer 20 is formed is a range beyond the first fold line F1 and the second fold line F2. The range in which the strength improving layer 20 is formed as illustrated in FIG. 13 is the same as the range illustrated in FIG. 6, and the same effect is obtained.

FIG. 13 illustrates a typical example of a tear TR when the individually packaged absorbent article 100 (FIG. 1) is unsealed. The tear (or fracture) TR is likely to occur, for example, in the vicinity of the first region R1 of the packaging sheet 10 where the fastening tape 30 is provided. The tear example TR is likely to occur in the packaging sheet 10 in the three-or-more-ply zone Za (FIGS. 1 and 4, etc.), and is likely to occur in the region R3a in the third region R3 particularly when the first region R1 is unfolded outside the longitudinal direction D1 and then the second region R2 is unfolded outside the longitudinal direction D1 and peeled from the third region R3. Although either the longitudinal direction D1 or the lateral direction D2 of the packaging sheet 10 may be the MD (or the CD) as described above, the MD of the paper is the lateral direction D2 and the CD is the longitudinal direction D1 in the example illustrated in FIG. 13. For this reason, as illustrated in FIG. 13, the tear example TR is likely to be formed substantially in the lateral direction D2. More specifically, a typical direction of progress of the tear example TR deviates slightly from the MD (lateral direction D2) and forms an angle in a range of more than 0° to 20° with respect to the lateral direction D2. Therefore, it is preferable that the strength improving layer 20 is formed so as to be orthogonal to the direction of development of the tear TR, because the action of preventing the progress of the tear would be improved. Therefore, for example, the angle formed by the extending direction of the linear portion included in the pattern of the strength improving layer 20 with respect to the lateral direction D2 is set preferably at 70° to 90°, and more preferably at 70° to less than 90°. More specifically, the angle α_{A} of the extending direction of the linear portions 20A, 20A, ... inclined to the right side in FIG. 13 with respect to the lateral direction D2 can be set at 70° to 90°, preferably at 70° to less than 90°, and similarly, the angle α_{B} of the extending direction of the linear portions 20B, 20B, ... inclined to the left side with respect to the lateral direction D2 can be set at 70° to 90°, preferably at 70° to less than 90°. Since both the linear portions 20A, 20A, ... and the linear portions 20B, 20B, ... are formed, it is possible to prevent both the right and left tears which are likely to occur in the region R3a of the third region R3.

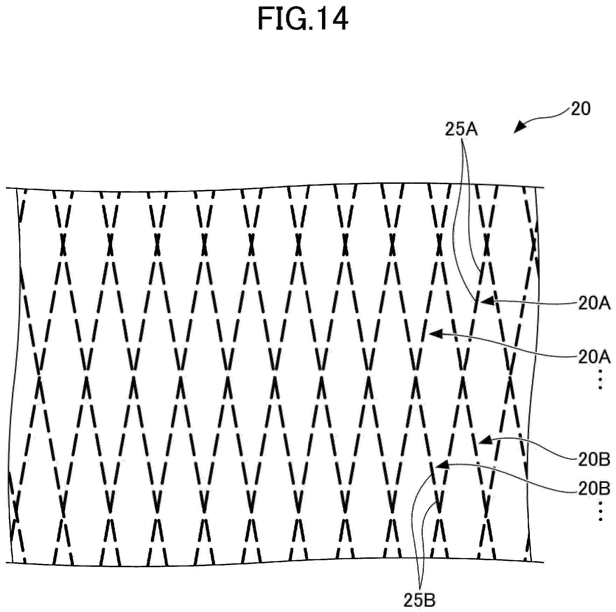

Further, FIG. 14 illustrates another modified example of the strength improving layer 20 of the packaging sheet 10 in the individually packaged absorbent article according to the fourth embodiment. In the example illustrated in FIG. 14, the strength improving layer 20 is also formed in a pattern including a plurality of linear portions, but each line is discontinuous. More specifically, the linear portion 20A is constituted by the linear portion elements 25A, 25A, ... which are spaced apart from each other, and the linear portion 20B is constituted by the linear portion elements 25B, 25B, ... which are spaced apart from each other. In this case, it is preferable that the interval between the linear portions arranged in a straight line is smaller than the length of the linear element. As illustrated in FIG. 14, the pattern in which the plurality of linear portions are formed of discontinuous linear portion elements is preferable from the viewpoint of saving the material of the strength improving layer and maintaining the original properties (ease of folding, texture, etc.) of the paper as much as possible, because the strength improving layer is arranged in a scattered manner.

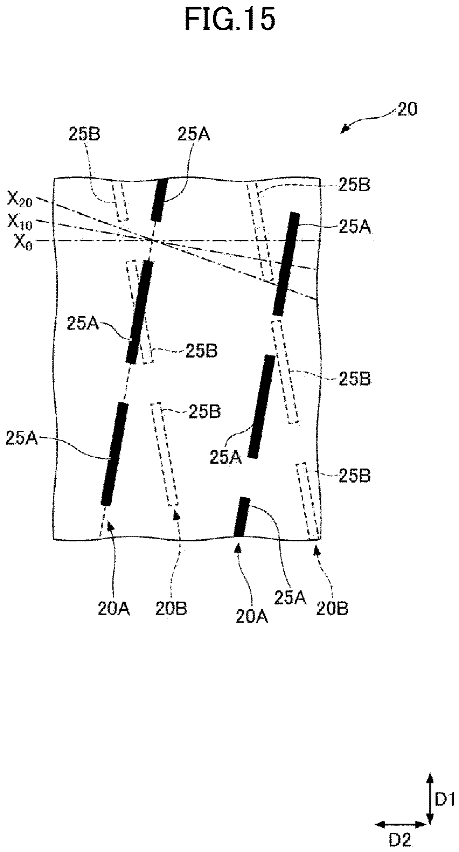

FIG. 15 illustrates still another modified example of the packaging sheet 10 in the individually packaged absorbent article according to the fourth embodiment. In FIG. 15, as in the example illustrated in FIG. 14, the pattern of the strength improving layer 20 includes a plurality of linear portions 20A, 20A, ... and 20B, 20B, ..., the linear portion 20A includes spaced linear portion elements 25A, 25A, ..., and the linear portion 20B includes spaced linear portion elements 25B, 25B, .... Herein, attention is paid to the linear portions 20A, 20A, ... having the same inclination (for the sake of attention, the linear portions 20B, 20B, ... are indicated by dotted lines in FIG. 15). As illustrated in FIG. 15, when the linear portions 20A, 20A, ... having the same inclination are compared with each other, the linear portion elements 25A, 25A, ... included in the linear portions 20A, 20A, ... may be arranged in a staggered manner.

Further, in view of the fact that the direction of the tear TR is 0° to 20° with respect to the MD (lateral direction D2), as illustrated in FIG. 15, when an arbitrary virtual straight line forming an angle of 0° to 20° with respect to the lateral direction D2 is drawn at an arbitrary position between the linear portion elements 25A, 25A included in one linear portion 20A of the adjacent linear portions 20A, 20A (that is, at an arbitrary place where the strength improving layer is not provided), the virtual straight line preferably intersects the linear portion elements 25A included in the other linear portion 20A. FIG. 15 illustrates, as examples of the virtual straight line, a virtual straight line X0 parallel to the lateral direction D2, a virtual straight line X10 forming an angle of 10° with respect to the lateral direction D2, and a virtual straight line X20 forming an angle of 20° with respect to the lateral direction D2. Thus, the packaging sheet 10 can be surely reinforced by the strength improving layer 20 with a small amount of material. Although the linear portions 20A, 20A, ... have been described with reference to FIG. 15, the same applies to the linear portions 20B, 20B, ....

### <Examples>

The tensile strength of the paper packaging sheet was measured in the MD and the CD.

### <Measurement of Tensile Strength>

A rectangular test piece that is a piece of the packaging sheet with a size of 120 mm in the MD × 25 mm in the CD was cut out from the packaging sheet. Both ends of the test piece in the MD were gripped by chucks (clamps) of a tensile tester ("Tensilon" manufactured by A & D Company Ltd.), and the test piece was pulled at a chucking interval of 50 mm and a tensile speed of 500 mm/min, and a load at the time of fracture (maximum load) was measured. The measurement was performed five times, and the arithmetic average was recorded as the MD tensile strength (N) of the packaging sheet to be tested.

A rectangular test piece that is a piece of the packaging sheet having a size of 25 mm in the MD × 120 mm in the CD was cut out from the packaging sheet, both ends of the test piece in the CD were gripped by chucks of the tensile tester, and the load was measured in the same manner as described above. The CD tensile strength (N) was determined in the same manner.

### (Example 1-1)

A packaging sheet was prepared by performing four color solid printing on the outer surface of 17 g/m² Unryu paper (manufactured by Maruishi Paper Tech Co., Ltd.) by flexographic printing.

### (Example 1-2)

A heat sealing agent ("Chemipearl (registered trademark) S500" manufactured by Mitsui Chemicals, Inc.) was applied to the inner surface of the paper packaging sheet of Example 1-1 by a flexographic printer so as to form a solid coating having a basis weight of 2.7 g/m².

Table 1 shows the measurement results of Example 1-1 and Example 1-2.

**[Table 1]**

| | Example 1-1 | Example 1-2 |
|---|---|---|
| Basis weight | 17 gsm | |
| Strength improving layer | None | Heat sealing agent coating (2.7 gsm) |
| MD tensile strength [N] | 29.16 | 38.85 |
| CD tensile strength [N] | 5.83 | 9.73 |

As shown in Table 1, it was found that the tensile strength was remarkably improved in both the MD and the CD by providing the strength improving layer (heat sealing agent).

### (Example 2-1)

A packaging sheet made of Unryu paper (manufactured by Maruishi Paper Tech Co., Ltd.) having a basis weight of 20 g/m² was prepared.

### (Example 2-2)

A PE film was extrusion laminated on the inner surface of the packaging sheet of Example 2-1 to a thickness of 6 µm.

### (Example 2-3)

A packaging sheet made of Unryu paper (manufactured by Maruishi Paper Tech Co., Ltd.) having a basis weight of 14 g/m² was prepared.

### (Example 2-4)

A PE film was extrusion laminated on the inner surface of the packaging sheet of Example 2-3 to a thickness of 8 µm.

Table 2 shows the measurement results of Example 2-1 through Example 2-4.

**[Table 2]**

| | Example 2-1 | Example 2-2 | Example 2-3 | Example 2-4 |
|---|---|---|---|---|
| Basis weight | 20 gsm | | 14 gsm | |
| Strength improving layer | None | 6 µm Laminate | None | 8 µm Laminate |
| MD tensile strength [N] | 29.11 | 32.53 | 16.87 | 26.2 |
| CD tensile strength [N] | 6.49 | 10.22 | 3.34 | 7.06 |

As shown in Table 2, it was found that the tensile strength was remarkably improved in both the MD and the CD by providing the strength improving layer (laminate of a resin film).

The present invention has been described above based on the embodiments, but the present invention is not limited to these embodiments. The above-described embodiments can be variously changed, modified, replaced, added, deleted, combined, and the like within the scope described in the claims, and these also belong to the technical scope of the present invention. The above-described components can be arbitrarily combined.

Preferred aspects of the present invention will be further described below.

A first aspect is an individually packaged absorbent article in which an absorbent article is individually packaged by a packaging sheet made of paper, wherein the packaging sheet is folded to an inner surface side in a first direction of the packaging sheet, both edge portions in a second direction orthogonal to the first direction are sealed, and a strength improving layer is provided on an inner surface and/or an outer surface of the packaging sheet.

The first aspect utilizes a packaging sheet made of paper. The paper-made packaging sheet has a natural and unique texture, and can give a sense of security, a sense of relaxation, a calm feeling, and the like to a user through the sense of sight, the sense of touch, and the like. In addition, the use of paper materials leads to a reduction in plastic materials, which is preferable from the viewpoint of achieving sustainable development goals.

Although a paper packaging sheet may not have a sufficient strength, the strength of the packaging sheet can be improved in the present aspect through the provision of a strength improving layer on the inner surface and/or the outer surface of the packaging sheet. Therefore, even in the case where a large force is locally applied to the packaging sheet, the packaging sheet can be prevented from being damaged. Therefore, the packaging sheet can be smoothly unfolded without being torn during unsealing. When an absorbent article is replaced, the used absorbent article can be wrapped with a packaging sheet that is not damaged or hardly damaged.

In a second aspect, the strength improving layer is formed by applying a strength improving agent, and the strength improving agent includes a heat sealing agent.

According to the second aspect, since the heat sealing agent is applied, heat sealing can be used for sealing both edge portions, and thus the sealing strength can be improved. This prevents the individually packaged absorbent article from being unintentionally unsealed before use, and provides an individually packaged absorbent article with high sealing properties.

In a third aspect, the packaging sheet has a basis weight of 8 g/m² to 50 g/m².

In the case where the packaging sheet is made of paper, it is preferable that the basis weight is relatively small in order to facilitate the process of folding or the like during the manufacture of the individually packaged absorbent article or from the viewpoint of suppressing a stiff feeling. According to the present aspect, such a preferable sheet can be obtained. However, since the strength of a packaging sheet having a smaller basis weight is usually lower, the packaging sheet may be damaged before or during unsealing or during the manufacture of an individually packaged absorbent article. Even in the case where a paper having a relatively small basis weight of 8 g/m² to 50 g/m² is used, a packaging sheet that is less likely to be torn can be provided by the strength improving layer described in the first aspect.

In a fourth aspect, the packaging sheet has a first region including one end in the first direction, a second region including the other end on the side opposite to the one end, and a third region between the first region and the second region, and is folded so that the first region and the second region overlap each other, and the strength improving layer is provided at least in one or more of a region of the first region overlapping the second region, a region of the second region overlapping the first region, and a region of the third region facing the portion where the first region and the second region overlap each other.

In the packaging form where the packaging sheet is folded into three or more sections, the sealing strength tends to be locally high in a zone where the number of layers of the packaging sheet is large (for example, a zone where the first region and the second region overlap each other); therefore, in that region, the packaging sheet is not smoothly peeled off when the seal is peeled off, and the packaging sheet is highly likely to be torn. In contrast, according to the fourth aspect, the strength improving layer is provided in the region corresponding to the region where the first region and the second region overlap each other, and thus the packaging sheet can be efficiently reinforced while the material of the strength improving layer is reduced.

In a fifth aspect, the basis weight of the strength improving layer provided in both edge portions of the packaging sheet is smaller than the basis weight of the strength improving layer provided in the region other than both edge portions.

In the case where the strength improving layer is provided by application of a heat sealing agent, heat sealing can be used to form the seal at the edges of the packaged absorbent article. Herein, in the case where the heat sealing agent is applied at a basis weight chosen in consideration mainly of improvement of the strength of the packaging sheet, the sealing strength at both edge portions may become excessively large. In this case, the operation of peeling off the packaging sheet at the time of unsealing does not proceed smoothly, and the possibility of tearing of the packaging sheet increases. In contrast, according to the fifth aspect, since the basis weight of the strength improving layer (heat sealing agent) differs between the edge portions and the region other than the edge portions, it is possible to prevent the sealing strength of the edge portions from being excessively increased, while the reinforcing function of the height improving layer (heat sealing agent) for the packaging sheet can be maintained.

In a sixth aspect, the strength improving layer is formed in a pattern including a plurality of linear portions in a plan view, and the linear portions extend at an angle of 70° to 90° with respect to the MD direction of the paper packaging sheet.

According to the sixth aspect, the strength improving layer is not continuously provided without a gap, but is patterned and formed so as to include a portion without the strength improving layer when viewed microscopically. Therefore, in the packaging sheet according to the present aspect, reinforcement by the strength improving layer is obtained, and the original bendability of the paper is maintained. Therefore, the folding process and the like at the time of packaging the individually packaged absorbent article are facilitated. Also, the patterned strength improving layer may reduce the amount of the material of the strength improving layer used.

In the case where a tear occurs in the packaging sheet, the tear usually tends to develop along the MD direction (the extending direction of the fibers) of the packaging sheet. Therefore, according to the present aspect, the strength improving layer is formed in a linear shape extending in a direction intersecting the MD, and thus it is possible to prevent the tear from developing. Therefore, a packaging sheet in which a tear is unlikely to develop can be provided with a small amount of the material of the strength improving layer.

This application claims priority based on Japanese Patent Application No. 2023-055947 filed on March 30, 2023, the entire contents of which are incorporated herein by reference.

### REFERENCE SIGNS LIST

1 Absorbent article
3 Top sheet
4 Absorber
7 Side sheet
10 Packaging sheet
11 One end in the longitudinal direction
12 Other end in the longitudinal direction
15 Sealing portion
20 Strength improving layer (strength improving agent)
30 Fastening tape
100, 100A Individually packaged absorbent article
D1 Longitudinal direction of the packaging sheet (first direction)
D2 Lateral direction of the packaging sheet (second direction)
F1 First fold line
F2 Second fold line
R1 First region
R2 Second region
R3 Third region
Za Thick zone (three-or-more-ply zone)
Zb Four-ply zone

## Claims

1. An individually packaged absorbent article in which an absorbent article is individually packaged by a packaging sheet made of paper, wherein
the packaging sheet is folded to an inner surface side in a first direction of the packaging sheet, and both edge portions in a second direction orthogonal to the first direction are sealed, and
a strength improving layer is provided on an inner surface and/or an outer surface of the packaging sheet.

2. The individually packaged absorbent article according to claim 1, wherein
the strength improving layer is formed by applying a strength improving agent, and
the strength improving agent includes a heat sealing agent.

3. The individually packaged absorbent article according to claim 1 or 2, wherein
a basis weight of the packaging sheet is 8 g/m² to 50 g/m².

4. The individually packaged absorbent article according to claim 1 or 2, wherein
the packaging sheet includes a first region including one end in the first direction, a second region including another end opposite to the one end, and a third region between the first region and the second region, and is folded in such a manner that the first region and the second region overlap each other, and
the strength improving layer is provided at least in one or more of a region of the first region overlapping the second region, a region of the second region overlapping the first region, and a region of the third region facing a portion where the first region and the second region overlap each other.

5. The individually packaged absorbent article according to claim 2, wherein
a basis weight of the strength improving layer provided in the both edge portions of the packaging sheet is smaller than a basis weight of the strength improving layer provided in a region other than the both edge portions.

6. The individually packaged absorbent article according to claim 1 or 2, wherein
the strength improving layer is formed in a pattern including a plurality of linear portions in a plan view, and
the linear portions extend at an angle of 70° to 90° with respect to a machine direction (MD) of the paper packaging sheet.
